# EUROPEAN PATENT APPLICATION

(11) **EP 1 704 778 A1**
(43) Date of publication of application: **27.09.2006**
(21) Application number: 05077830.7
(22) Date of filing: 09.12.2005
(51) Int. Cl.: A01K 67/027, C12N 15/85, C12N 5/10, C07K 14/47

(54) **FO66 transgenic mice that express pathogenic mutants of the sca2 gene**

(30) Priority: 09.12.2004 CU 27804
(71) Applicant: CENTRO DE INGENIERIA GENETICA Y BIOTECNOLOGIA (CIGB), Ciudad de la Habana 10600 (CU)
(72) Inventor: Aguiar Santiago, Jorge Agustin, Vibora, Ciudad de La Habana (CU); Fernandez Masso, Julio Raúl, Cubanacán, Playa C. de La Habana (CU); Mendoza Mari, Yssel, Habana Vieja Ciudad de La Habana (CU); Vasquez Marcos, Maria Mercedes, 11600 (CU); Cruz Leo, Silian, C. de La Habana (CU); Velazquez Perez, Luis Clodoaldo, Rpto Santiesteban 80632 (CU); Herrera Martinez, Luis Saturnino, Ciudad de La Habana (CU)
(74) Representative: Winckels, Johannes Hubertus F.

(57) **Abstract**

The present invention is related with the neurobiology and the transgenic animals, more specifically with obtaining a model to study the SCA2 genetic disease. The genome of one mice line has been modified through the introduction of a DNA segment that contains the necessary information for the synthesis of the human ataxin 2. In particular the invention is related with the transgenic mice F066 and the cellular lines able to express the human sca2 gene under the regulation of its self promoter. The F066 transgenic mice reproduce the features of the disease.

The homozygous transgenic mouse line for the new gene was obtained through matting. This transgenic line is useful to study the mechanism that produce the disease and also will permit to prove new therapeutics that contributes to minimize the clinical symptoms in the patients.

## Description

The present invention is related with the neurobiology and the transgenic animals, more specifically with the obtaining and the methods to use an animal model to study the genetic disease spinocerebellar ataxia type 2 (SCA2). Its essence consist in that the genome of one mice line has been modified through the introduction of a deoxyribonucleic acid (DNA) segment that contain the necessary information for the synthesis of the human ataxin 2. In particular the invention is related with the transgenic mice F066 and the self derived cellular lines that will be able to express the human sca2 gene under the regulation of its self promoter. Those transgenic mice, named F066, reproduce some of the symptoms of the SCA2 disease.

The dominant spinocerebellar ataxias are a neurodegenerative disease group that affects mainly the Purkinje neurons of the cerebellum. The spinocerebellar ataxia type 2 (SCA2) is an autosomic dominant genetic disease characterized by the gait ataxia, difficulties in speech, abnormal behavior of the reflexes, and slow and limited eye movements (Velázquez L, Medina HE (1999). Neurophysiologic evaluation in patients affected by spinocerebellar ataxia type 2. *Rev. Neurol* 27 (160): 921-926).

Molecularly, the appearance of the symptoms of the disease its produce when the pathogenic allele present 32 or more CAG repeats, and these repeats are considered as expansions (Santos et al (1999). Molecular diagnosis of a sample of the Cuban population with spinocerebellar ataxia type 2. *Biotecnología Aplicada* 16: 219-221). The beginning of the disease symptoms correlate inversely to the length of the expansion. The disease is progressive and patients die 15-18 years after symptom onset. The more frequently pathogenic repeat is 37 CAG or glutamines. The number of glutamine repeats is associated with the age of clinic symptoms onset from 20 to 60 years (Santos et al (1999). Molecular diagnosis of a sample of the Cuban population with spinocerebellar ataxia type 2. *Biotecnología Aplicada* 16: 219-221).

The neuropathologic studies in death SCA2 patients show a marked reduction in the Purkinje cells number with poor dendrite arborization. There is also a decrease in the number of granule cells, the inferior olive neurons and the neurons from the pontocerebellar nuclei. There is also, a marked reduction of the neurons from the nigral substance, desmienilization of the spinal cord and reduction of the number and size of the motor neurons (Estrada R et al (1999) Spinocerebellar ataxia 2 (SCA2): morphometric analyses in 11 autopsies. *Acta Neuropathol (Berl)* 97(3): 306-310)

The molecular studies have been demonstrated that normal ataxin 2 is a 140 kDa protein ubiquitously expressed in the human and mice tissues. The messenger RNA in humans is localized in the brain, heart, placenta, liver, muscle and pancreas with little expression in kidney and lung. Different to other polyglutamine diseases the formation of nuclear aggregates is not associated with the pathogenic mechanism (Pulst et al (1996). Moderate expansion of a normally biallelic trinucleotide repeat in spinocerebellar ataxia type 2. *Nature Genet* 14: 269-276).

The degenerative process of Huntington and SCA diseases are very difficult to elucidate and very aggressive. The identification of the mutant genes in these diseases has permitted the determination of the pathogenesis to a basic level. Recently, studies in transgenic mouse models and patients have permitted to know some of the molecular changes that conduce to neuronal dysfunction and death in the polyglutamine diseases. At the same time, the appearance of mutant mice that show neurological phenotypes have permitted to discover other type of secondary neurodegenerative diseases like the alteration in the ionic canals or the alteration in the neurotransmitter receptor functions (Heintz N, Zoghbi Y (2000). Insights from mouse models into the molecular basis of neurodegeneration, *Annu. Rev. Physiol* 62: 779-802).

Although the studies in normal and disease human brains could bring important aspects to the knowledge of the pathogenesis of the diseases caused by polyglutamine expansions, some observations are limited to the terminal steadies of the disease. The transgenic models could circumvent this problem, but many of then are constructed using truncated constructs with large polyglutamine tracts to produce neurodegeneration. Beside, a great number of these models don't show high neuronal loss, feature that define the polyglutamine diseases in humans (Davies et al 1999). From neuronal inclusions to neurodegeneration: neuropatological investigation of a transgenic mouse model of Huntington's disease. *Phil. Trans. R.Soc. Lond. B Biol.* Sci. 234: 981-989).

In the US patent 6,515,197 it is describe a transgenic SCA2 model developed by (Huynh et al (2000). Nuclear localization or inclusion body formation of ataxin-2 is not necessary for SCA2 pathogenesis in mouse or human, *Nature Genetics* 26: 1-7). These animals show ataxin 2 accumulation in the cytoplasm and symptoms of the SCA2 disease, but its limitations are the transgene expression and the pathogenic protein production only in the Purkinje cells. Also, the relatively few number of CAG/polyglutamine repeats of the pathogenic allele (58 CAG-58 Q) from these transgenic mice produce deficiencies in the study of the protein effects in the cell lines derived of this model, because the cells don't show clear phenotypic effects.

In consequence, in this field it's imposing the expression of the SCA2 gene regulated under its self promoter. The transgenic animals generated with this construction will express the heterologous gene not only in the Purkinje cell, also will simulate the expression patterns of the SCA2 gene in other cells and tissues like occur in humans. Additionally, the use of a larger expansion (75 CAG-75 Q) brings more possibilities of the obtaining of cell lines derived of the transgenic mice with evident phenotypic effects.

### Detailed description of the invention

The present invention show a useful transgenic mouse model to study the genetic disease spinocerebellar ataxia type 2, where the mouse it's characterize by the human SCA2 gene expression regulated by it's self promoter, and also it's capable of reproduce some of the features of the human disease. The transgenic mice could be homozygous or heterozygous for the transgene.

It is a general objective of the present invention to produce transgenic mice or cell lines derived from them, that contain one or two copies of the human SCA2 gene with 75 CAG repeats integrated in the genome.

An aspect of the present invention is to proportionate a method for the screening of different biological or chemical agents capable to modulate the associated phenomena with the neurodegeneration, for example the motor in-coordination, the apoptosis and the formation of polyglutamine aggregates, whre the method involucrate the steps to combine a candidate substance with the transgenic mouse and the evaluation of his effects in the model. These substances could be short chemical molecules or endogenous or exogenous biological molecules useful for the prevention, the treatment, or the diminution of the clinical symptoms of the disease. Another objective is to proportionate a murine model of the study of the genetic disease spinocerebellar ataxia type 2, that permit the study of the progress of the disease and the associated symptoms, and the identification of the prevention mediums.

In another aspect of the invention, the transgenic mice could be additionally manipulate to permit the expression of other genes that could have therapeutic effects like for example growth factors like IGF-1, NGF, BDNF, and anti-apoptotic genes like Bcl-2 and others.

### Transgenic animals

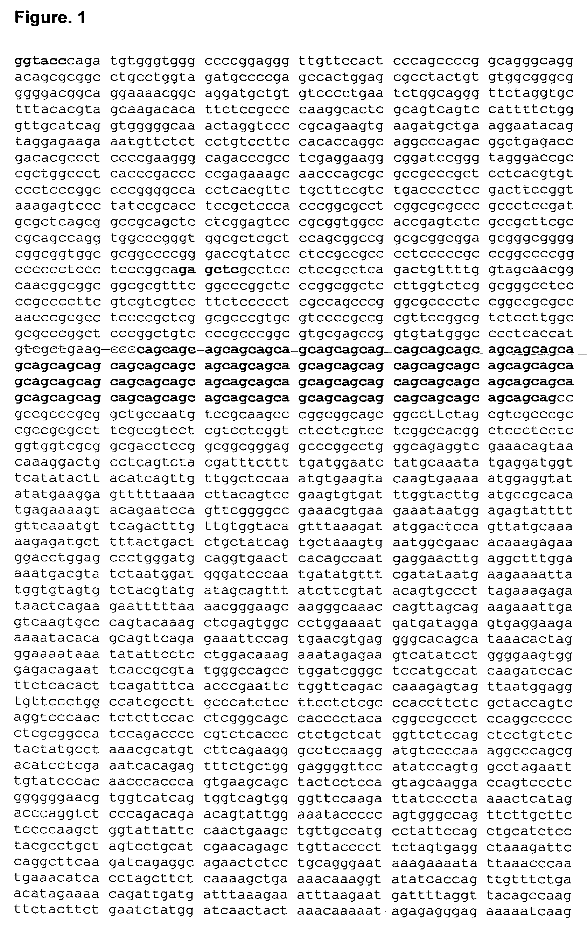

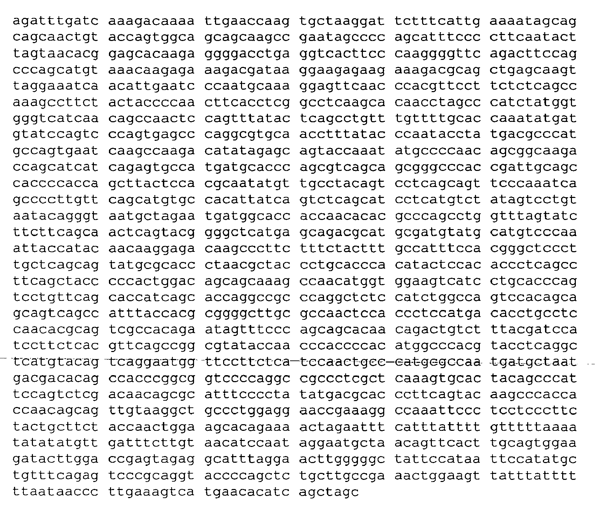

In the present invention the exogenous gene is united operationally to the minimal promoter region of the human SCA2 gene (figure 1). This promoter is functional in several cellular lines of different origin and specie (Aguiar et al (1999). Cloning and sequence of the 5' region from human spinocerebellar ataxia 2 gene. *Biotecnología Aplicada* 16: 165-168, Aguiar et al (1999). Identificaction of the physiological promoter for spinocerebellar ataxia type 2 reveals a CpG island for promoter activity situated into the exon 1 of this gene and provides data about the origin of the nonmethylated state of these types of islands, *Biochem. Biophys. Res. Commun.* 254: 315-318).

The transgene contains a pathological expansion of 75 CAG repeats, one of the largest reported for a Cuban patient. This patient has a disease onset at 6 years old. The gene could be manipulate with the objective of introduce deletions, substitutions or insertions like for example to increase the number of CAG repeats. The promoter region could be modified with the objective to regulate or increase the level of the SCA2 gene expression with pathological CAG repeats. The transgenic mouse of the present invention could receive other alterations like for example to eliminate the function of the endogenous gene.

### Nuclei acid composition

The constructions for his use in the present invention could include any construction appropriate for the generation of transgenic animals that express the SCA2 gene or fragments of this gene that contains 75 or more CAG repeats operated under the regulation of his self promoter in the different cells and tissues like occur in humans. These constructions could contain cDNA, genomic sequences or both.

The methods for the isolation and cloning of the target sequences from different sources and the constructions for his expression are reported in the literature. Besides, the constructions could contain reporter genes like Lac Z, under the control of the human SCA2 promoter to facilitate the detection of the expression. It is possible to generate protein fusion or other modifications that permit to make localization studies in tissues or sub-cellular regions. Other mutations could be use to make structure- function studies.

### Other human genes

There are a number of human genes involved in neurodenegeration mechanisms. Transgenic animals in the present invention could carry aditional genes that accelerate neurodegeneration processes, neuronal plasticity or mediate neuroprotection.

### Drug screening assays

It is possible to identify ligands or substrates that mimic neurodegeneration processes using transgenic animals or their derived cell lines expressing pathogenic variants of sca2 gene. Such compounds could act either directly on ataxin 2 or on mechanisms associated to the disease. Thus, identified therapeutical targets and designed drugs derived from the study of these transgenic animals could be useful for other CNS pathologies since they share common neurodegenerative mechanisms, including insoluble aggregates formation and apoptosis activation. That is the case of low toxicity drugs screening.

A wide number of assays can be use in this way, for example motor behaviour evaluation, immunenzymatic and molecular biology assays, neurophysiological evaluation, and localization of drugs. Mice or mice-derived cells could be useful depending on the assay. Cells can be extracted from animals or immortalized. Central Nervous System derived cells are of particular interest, specifically Purkinje cells. Besides, other cell types, for example bone marrow stromal cells.

Therapeutical candidates include a group of chemical compounds, tipically low weight organic molecules, an also biomolecules, such as peptides, carbohydrates, fatty acids, steroids, lipids and their derivatives or combinations. Candidates can be obtained from different sources, including libraries containing large numbers of natural and synthetic compounds. Those compounds can be modify to produce structural analogs with improved pharmacological properties. Among therapeutical agents are bone marrow stromal cells, which can be derived from transgenic or wild type mice. These cells can be genetically engineered and systemically or stereotactically administered directly into the lession site. Screening can be managed to pharmacologically well known active compounds or their analogs or derivatives. To obtain therapeutical effects candidates can be formulated and administered with a pharmaceutically appropriate carrier.

High throughput *in vivo* screening usually includes the use of a number of animals receiving different concentrations of therapeutical candidate. It also includes different formulations of the same candidate. These agents can be administered in one dose only combined with other molecules when it is possible to obtain a synergic effect. Monitoring the effect ca be carried out by conventional methodologies.

### Description of the drawings

Figure 1. Sequence of the sca2 human promoter fused to the sca2 human gene with 75 CAG repeats (total length of 5.017 kbp). In bold, restriction sites for Kpnl (ggtacc) and Sacl (gagctc), flanking human sca2 gene promoter (0.8 kbp) (Aguiar et al. 1999. *Biochem. Biophys. Res. Commun.* 254: 315-318). Within sca2 cDNA sequence (4.217 kbp), in bold 75 CAG region, isolated from a Cuban patient.
Figure 2. Schematic representation of DNA fragment for microinjection (5.285 kbp): human sca2 gene promoter (0.8 kbp), human sca2 cDNA (4.217 kbp) with 75 CAG repeats, SV40 polyA signal (0.268 kbp).
Figure 3. Transgen construction design.
Figure 4. Polymerase chain reaction on chromosomal DNA.
   (A) 1-3: F0 sca2-75CAG transgenic mice; 4: wild type mouse; 5: molecular weight marker BlueScript/Hpall, 6: control plasmid for 22 CAG repeats, 7: control plasmid for 75 CAG repeats, 8: water as negative control.
   (B) 1-6: F2 sca2-75CAG transgenic mice, 7: wild type mouse, 8: cuban patient carrying 22 and 41 CAG repeats, 9: control plasmid for 75 CAG repeats, 10: molecular weight marker BlueScripVHpall.

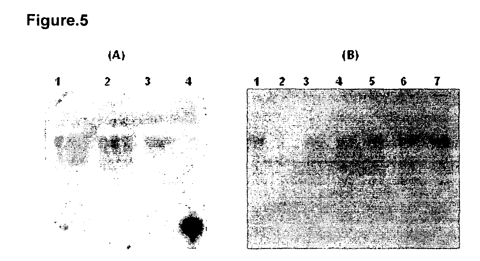

Figure 5. Southern blot.
   (A) Sacl-EcoRl fragment hybridization. 1-2: F0 sca2-75CAG transgenic mice, 3: wild type mouse, 4: 0.1 ng of control plasmid for 75 CAG repeats, Sacl-EcoRl digested.
   (B) Kpnl fragment hybridization. 1: 0.01 ng of control plasmid for 75 CAG repeats, Kpnl digested, 2: wild type mouse, 3-7 F2 sca2-75CAG transgenic mice.

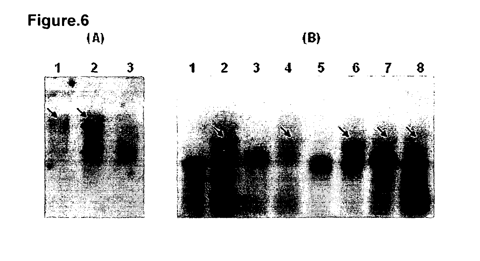

Figure 6. Transgene expression analysis in different tissues of transgenic mice by Northern blot. Age-paired wild type animals as negative controls. Molecular weight for human sca2-75CAG transgene (4 kbp) is indicated by arrows.
   (A) Cerebellum: 1- F2 transgenic mouse, homozygous for sca2-75CAG human gene. 2- F1 transgenic mouse, heterozygous for sca2-75CAG human gene. 3- Wild type mouse.
   (B) 1, 3, 5- Brain, liver and skeletal muscle from wild type mouse. 2, 4, 6, 7, 8 - Brain, liver, skeletal muscle, lung and kidney from heterozygous transgenic mouse.

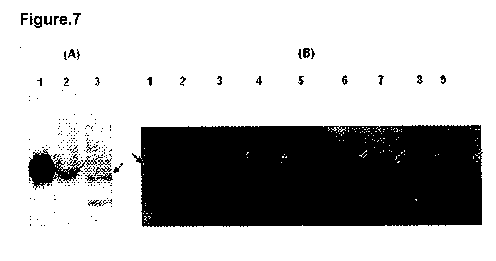

Figure 7. Transgene expression analysis in different tissues of transgenic mice by Western blot. Age-paired wild type animals as negative controls. Western blots were carried out using a monoclonal antibody raised against the N-terminal fragment of the human ataxin 2 protein, which includes ATG and a 22 CAG repeats region. Arrows point to wild type ataxin 2 protein (140 KD). Higher molecular weight proteins could be expanded ataxin 2 protein with 75 CAG repeats or ataxin 2 protein aggregated to different proteins. In samples corresponding to transgenic mice there are strongly stained lower weight proteins, which could be degradation fragments of expanded ataxin 2 with 75 CAG repeats.
   (A) 1- Monoclonal antibody (150 KD) as molecular weight marker. 2- Cerebellum from wild type mouse. 3- Cerebellum from heterozygous sca2-75CAG transgenic mouse.
   (B) 1- Cerebellum from wild type mouse. 2- Monoclonal antibody (150 KD) as molecular weight marker. 3-9: Cerebellum, brain, liver, heart, skeletal muscle, kidney and lung from heterozygous sca2-75CAG transgenic mouse.

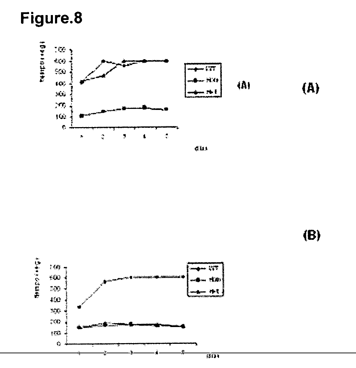

Figure 8. Rotarod test's results from two sca2-75CAG transgenic mice and one age-paired wild type mouse. The graph shows average performance on Rotarod apparatus of 4 trials each day on 5 consecutive days.
   (A) Six weeks. Homozygous mice show clear symptoms of motor impairment compared to wild type.
   (B) Twelve weeks. Both, homozygous and heterozygous transgenic mice show motor impairment compared to wild type.

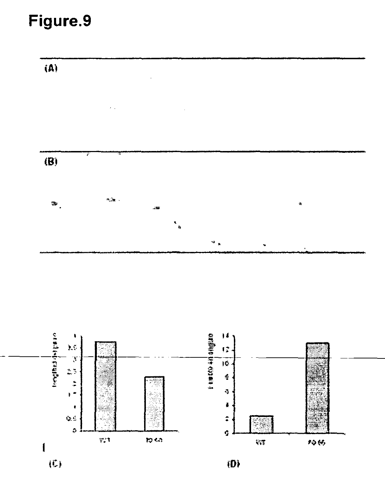

Figure 9. Analysis of footprint patterns of wild type mouse (A) and heterozygous F0 transgenic mouse (B). Quantitative comparison of step length (C) and linearity of the movements (D).

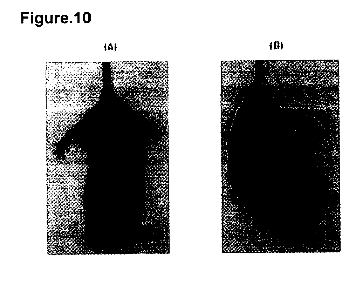

Figure 10. Clasping posture. The wild type mouse extends the back legs as a classical posture in order to steady itself (A). An heterozygous transgenic mouse binds the back legs demonstrating the feet-clasping posture when suspended by the tail (B).

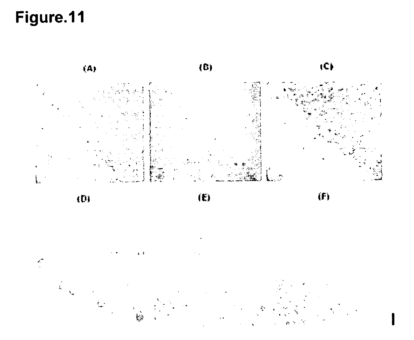

Figure 11. Anatomical changes evaluation. (A, B, C) Cresyl Violet staining. (D, E, F) Anti-calbindin 28K immunhistochemistry. (A, D) Cerebellum from one year old wild type mouse. (B, C, E, F) Cerebellum from one year old heterozygous sca2-75CAG transgenic mouse.

### Example 1

### Obtention and molecular characterization of F0 transgenic mice carrying sca2-75CAG human gene.

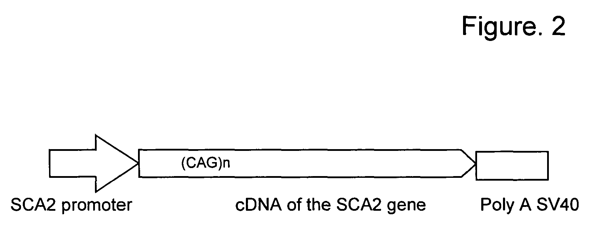

The genetic construction is composed by the following elements 5'-3' ordered (Figure 2):
a) A 0.8 kbp fragment encoding human ataxin 2 gene promoter (Aguiar et al (1999) Cloning and sequence of the 5' region from human spinocerebellar ataxia 2 gene. Biotecnología Aplicada 16: 165-168, Aguiar et al (1999). Identification of the physiological promoter for spinocerebellar ataxia type 2 reveals a CpG island for promoter activity situated into the exon 1 of this gene and provides data about the origin of the nonmethylated state of these types of islands, Biochem. Biophys. Res. Commun. 254: 315-318).
b) A 4.217 kbp fragment encoding complementary DNA for sca2 gene carrying 75 CAG repeats. This fragment was obtained from a patient blood total RNA sample by RT-PCR.
c) A 0.268 kbp fragment encoding transcription termination and polyadenylation signals from SV40 virus, present in pGL3-Luc Basic Vector (Promega) plasmid.

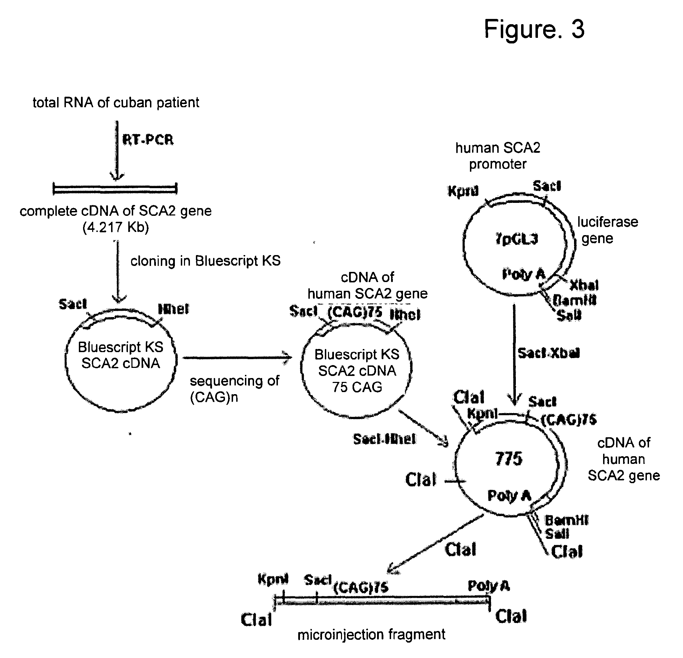

Figure 3 schematically represents the genetic construction used to generate transgenic mice line. Kpnl-Sacl fragment containing sca2 gene promoter region was isolated from a genomic clone and was inserted into pGL3-Luc Basic Vector (Promega). The sca2 cDNA carrying 75 CAG repeats was isolated by RT-PCR from a cuban patient. This fragment was cloned into pBlueScript KS (Stratagene) plasmid.

The late expression genes 3' region from SV40 virus carrying transcription termination and polyadenylation signals was obtained from pGL3-Luc Basic Vector (Promega) plasmid.

Final genetic construction was made following standard procedures described by Sambrook et al. (Sambrook et al (1982). Molecular cloning-a laboratory manual. 2nd Ed. Cold Spring Harbor Laboratory Press). Final plasmid was identified as 775. For transgenesis final construction was Clal digested, to obtain the fragment carrying promoter, cDNA with 75 CAG repeats and SV40 signals encoding regions. Transgenic animals were obtained by previously described methods (Castro FO, Aguilar A (1989). Microinjection, culture, and transfer of one cell mouse embryos. I- Obtention of one cell embryos and its culture. *Interferón y Biotecnología* 6: 65-70, Castro FO, Aguilar A (1989). Microinjection, culture and transfer of one cell mouse embryos. II-Microinjection, culture and transfer of one cell mouse embryos (in Spanish). *Interferón y Biotecnología* 6: 172-174, Castro FO, Aguilar A (1992). Effect of the number of transferred embryos microinjected or non-manipulated on the pregnancy rate and litter size in B6D2F1 mice. *Theriogenology* 36:105). Mice strain used was a hybrid B6D2F1 X OF1, obtained in our laboratory. Parent mice strains B6D2F1 and OF1 were obtained from National Center for the Production of Laboratory Animal, Havana, Cuba.

Transgenic mice were obtained by microinjection of 1-2 picolitres of buffer solution containing 2 ng per microliter linear DNA fragment into one of the unicellular embryo pronuclei of mouse. Embryos were transferred to recepter female mice and, after birth, littermates were analyzed to detect the presence of transgene by Polymerase Chain Reaction (PCR) (Saiki et al (1989). Primer directed amplification of DNA with a thermostable DNA polymerase. Science 239: 487-491).

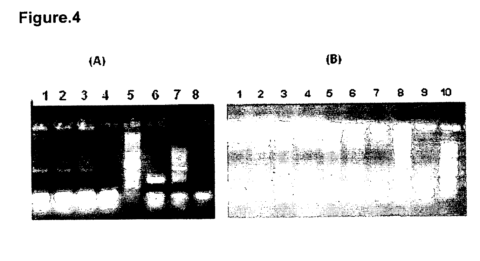

From a total of 71 littermates, 15 were positive for the presence of the transgene (Figure 4), 3 males and 12 females. DNA hybridization techniques allowed us to corroborate PCR results and to determine the number of copies integrated into transgenic mice genome (Sambrook et al (1982). Molecular cloning-a laboratory manual. 2nd Ed. Cold Spring Harbor Laboratory Press). Homozygous animals were obtained by crossing. It was determined by DNA hybridization techniques that the progeny of homozygous mice carry one or two copies of the transgene, and that it is transmitted to the next generation according to Mendel genetic laws. Human transgene expression is ubiquitous in transgenic mice inside and outside Central Nervous System, according to Northern (Figure 6) and Western blot (Figure 7) results.

### Example 2

### Functional Testing of Transgenic Mouse Line F066 by Rotarod

This example describes functional characterization of SCA2 transgenic mouse lines by Rotarod. Motor coordination performances of wild type, homozygous and heterozygous mice were tested using Basile Rotarod treadmills. The duration of the test was 5 days with 4 individual trials Mice were placed on an accelerating rod with a rotating speed from 4 to 40 r.p.m. for 300 seg and with a constant speed of 40 rpm until 600 seg with a resting time of 15 min, between each test. An example of mice performance on the test is represented in figure 8. The graph shows average performance on the Rotarod apparatus of four trials each day on five consecutive days. Transgenic mice fall early from Rotarod than wild type mice, showing motor deficit. Differences were significant for homozygous at 8 weeks, and for heterozygous mice at 16 weeks. Rotarod has been used extensively to assess functional impairment in SCA2 transgenic animals. First it was used to evaluate the 15 F0 positive by PCR animals, then it was used to evaluate the F1, F2 and homozygous progenies.

### Example 3

### Evaluation of homozygous animals derived from the F066 mice.

The homozygous mice derived from the FO66 mice, were obtained by matting the founder FO66 mice and a transgenic F1 animal. The homozygous character was evaluated by matting with wild type animals. The progenies derived from the matting produced 100% heterozygous animals. The result was demonstrated by PCR analysis of F3 descendents. After analysis of DNA by PCR all the animals were positive for the presence of the ADNc-SCA2-75CAG transgene. The motor deficit was also demonstrated in all animals derived from a homozygous parent. The progenies derived from four generations keep the same morphological, reproductive and conduct phenotype as the parental animals.

### Example 4

### Footprinting analysis of transgenic line F066

Mouse was allowed to run through a dark 30 cm long, 10 cm wide and 6cm height tunnel. To measure stride length, the back foots were painted with black aqueous ink. The direction of foot printing was also evaluated. The angle difference between two consecutive steps was calculated. The absolute value of angles was expressed as the sum of angles differences divided by the number of steps. The total stride length value obtained from each animal was an average of the stride length from the total number of steps produced by each leg. Footprint analysis showed progressive reduction in stride length for the transgenic animal compared with the wild type. A typical result is shown in figure 9.

### Example 5

### Clasping evaluation of the F066 transgenic line.

Mice with neurodegenerative phenotypes have a tendency to fold their hindlegs when held by the tail for at least one minute. For the clasping test, mice were held by the tail for one minute. Figure 10 shows the clasping observed in transgenic animals derived from the F066 mice. No clasping was observed in wild type animals from the same age.

### Example 6

### Anatomical changes observed in the transgenic line F066

This example describes morphological characterization of SCA2 transgenic mouse lines. Transgenic and control wild type mice from the same age were sacrificed.

Brains were removed and embedded in paraffin. Tissue sections were stained with cresyl violet to evaluate the anatomical differences between tissues. Transgenic F066 animals showed a gradual Purkinje cell loss (figure 11, B y C). To investigate morphological changes in Purkinje cells, calbindin-28K immunoreactivity in tissue sections from heterozygous animals was compared with that from wild-type mice.

Calbindin-28K is a protein specifically expressed in cytoplasm and dendritic processes of cerebellar Purkinje cells. Changes in calbindin-28K labeling were seen in young animals but became more pronounced with age. Most Purkinje cells lost calbindin-28K immunoreactivity in transgenic mice from line F066 . Loss of the dendritic arbour was also observed closely followed by a loss in Purkinje cell number (figure 11, E y F). These results show morphological neurodegenerative phenotypes are exhibited in transgenic mice expressing human SCA2 having a 75 polyglutamine repeat under the control of the human SCA2 promoter.

## Claims

1. A transgenic mouse named FO66, containing integrated in the genome a DNA sequence identified in the sequence listing as Seq.ID. 1, comprising a polyglutamine tract of about 75 CAG in the first exon of the ataxin-2 polypeptide, said sequence operatively linked to a SCA2 human promoter that reproduce the expression pattern observed in human patients.

2. The transgenic mouse of claim 1, wherein said mouse is homozygous for said transgene.

3. The transgenic mouse of claim 1, wherein said mouse is heterozygous for said transgene.

4. The progenies of transgenic mouse of claim 1, 2 y 3, **characterized by** the same morphological, reproductive and conduct characteristics as the parental animals

5. The use of the transgenic animal of claims 1, 2, 3, y 4, for the generation of cellular lines containing as part of their genome the sequence identified as Seq.ID. 1, with an expansion of 75 CAG in the first exon of the SCA2 gene, said sequence operatively linked to a SCA2 human promoter.

6. Cellular lines according to claim 5, containing as part of their genome the sequence identified as Seq.ID. 1, with an expansion of 75 CAG in the first exon of the SCA2 gene, said sequence operatively linked to a SCA2 human promoter.


### Annex to the application documents - subsequently filed sequences listing

SEQUENCE LISTING

<110> Centro de Ingenieria Genetica y Biotecnologia

<120> F066 transgenic mice that express pathogenic mutants of the sca2
gene

<130> P75573EP00

<140> EP 0507783.7
<141> 2005-12-09

<150> 2004-0278
<151> 2004-12-09

<150> CU 2004-0278
<151> 2004-12-09

<160> 1

<170> PatentIn version 3.3

<210> 1
<211> 5017
<212> DNA
<213> Artificial Sequence

<220>
<223> Pathogenic mutant of human SCA2 gene exon 1

<220>
<221> promoter
<222> (1)..(800)
<223> SCA2 promoter

<220>
<221> exon
<222> (800)..(5017)
<223> SCA2 cDNA

<400> 1
ggtacccaga tgtgggtggg ccccggaggg ttgttccact cccagccccg gcagggcagg 60

acagcgcggc ctgcctggta gatgccccga gccactggag cgcctactgt gtggcgggcg 120

ggggacggca ggaaaacggc aggatgctgt gtcccctgaa tctggcaggg ttctaggtgc 180

tttacacgta gcaagacaca ttctccgccc caaggcactc gcagtcagtc cattttctgg 240

gttgcatcag gtgggggcaa actaggtccc cgcagaagtg aagatgctga aggaatacag 300

taggagaaga aatgttctct cctgtccttc cacaccaggc aggcccagac ggctgagacc 360

gacacgccct ccccgaaggg cagacccgcc tcgaggaagg cggatccggg tagggaccgc 420

cgctggccct cacccgaccc ccgagaaagc aacccagcgc gccgcccgct cctcacgtgt 480

ccctcccggc cccggggcca cctcacgttc tgcttccgtc tgacccctcc gacttccggt 540

aaagagtccc tatccgcacc tccgctccca cccggcgcct cggcgcgccc gccctccgat 600

gcgctcagcg gccgcagctc ctcggagtcc cgcggtggcc accgagtctc gccgcttcgc 660

cgcagccagg tggcccgggt ggcgctcgct ccagcggccg gcgcggcgga gcgggcgggg 720

cggcggtggc gcggccccgg gaccgtatcc ctccgccgcc cctcccccgc ccggccccgg 780

cccccctccc tcccggcag agc tcg cct ccc tcc gcc tca gac tgt ttt ggt 832
Ser Ser Pro Pro Ser Ala Ser Asp Cys Phe Gly
1               5                   10

agc aac ggc aac ggc ggc ggc gcg ttt cgg ccc ggc tcc cgg cgg ctc 880
Ser Asn Gly Asn Gly Gly Gly Ala Phe Arg Pro Gly Ser Arg Arg Leu
            15                  20                  25

ctt ggt ctc ggc ggg cct ccc cgc ccc ttc gtc gtc gtc ctt ctc ccc 928
Leu Gly Leu Gly Gly Pro Pro Arg Pro Phe Val Val Val Leu Leu Pro
        30                  35                  40

ctc gcc agc ccg ggc gcc cct ccg gcc gcg cca acc cgc gcc tcc ccg 976
Leu Ala Ser Pro Gly Ala Pro Pro Ala Ala Pro Thr Arg Ala Ser Pro
    45                  50                  55

ctc ggc gcc cgt gcg tcc ccg ccg cgt tcc ggc gtc tcc ttg gcg cgc 1024
Leu Gly Ala Arg Ala Ser Pro Pro Arg Ser Gly Val Ser Leu Ala Arg
60                  65                  70                  75

ccg gct ccc ggc tgt ccc cgc ccg gcg tgc gag ccg gtg tat ggg ccc 1072
Pro Ala Pro Gly Cys Pro Arg Pro Ala Cys Glu Pro Val Tyr Gly Pro
                80                  85                  90

ctc acc atg tcg ctg aag ccc cag cag cag cag cag cag cag cag cag 1120
Leu Thr Met Ser Leu Lys Pro Gln Gln Gln Gln Gln Gln Gln Gln Gln
            95                  100                 105

cag cag cag cag cag cag cag cag cag cag cag cag cag cag cag cag 1168
Gln Gln Gln Gln Gln Gln Gln Gln Gln Gln Gln Gln Gln Gln Gln Gln
        110                 115                 120

cag cag cag cag cag cag cag cag cag cag cag cag cag cag cag cag 1216
Gln Gln Gln Gln Gln Gln Gln Gln Gln Gln Gln Gln Gln Gln Gln Gln
    125                 130                 135

cag cag cag cag cag cag cag cag cag cag cag cag cag cag cag cag 1264
Gln Gln Gln Gln Gln Gln Gln Gln Gln Gln Gln Gln Gln Gln Gln Gln
140                 145                 150                 155

cag cag cag cag cag cag cag cag cag cag cag cag cag cag cag cag 1312
Gln Gln Gln Gln Gln Gln Gln Gln Gln Gln Gln Gln Gln Gln Gln Gln
                160                 165                 170

cag cag ccg ccg ccc gcg gct gcc aat gtc cgc aag ccc ggc ggc agc 1360
Gln Gln Pro Pro Pro Ala Ala Ala Asn Val Arg Lys Pro Gly Gly Ser
            175                 180                 185

ggc ctt cta gcg tcg ccc gcc gcc gcg cct tcg ccg tcc tcg tcc tcg 1408
Gly Leu Leu Ala Ser Pro Ala Ala Ala Pro Ser Pro Ser Ser Ser Ser
        190                 195                 200

gtc tcc tcg tcc tcg gcc acg gct ccc tcc tcg gtg gtc gcg gcg acc 1456
Val Ser Ser Ser Ser Ala Thr Ala Pro Ser Ser Val Val Ala Ala Thr
    205                 210                 215

tcc ggc ggc ggg agg ccc ggc ctg ggc aga ggt cga aac agt aac aaa 1504
Ser Gly Gly Gly Arg Pro Gly Leu Gly Arg Gly Arg Asn Ser Asn Lys
220                 225                 230                 235

gga ctg cct cag tct acg att tct ttt gat gga atc tat gca aat atg 1552
Gly Leu Pro Gln Ser Thr Ile Ser Phe Asp Gly Ile Tyr Ala Asn Met
                240                 245                 250

agg atg gtt cat ata ctt aca tca gtt gtt ggc tcc aaa tgt gaa gta 1600
Arg Met Val His Ile Leu Thr Ser Val Val Gly Ser Lys Cys Glu Val
            255                 260                 265

caa gtg aaa aat gga ggt ata tat gaa gga gtt ttt aaa act tac agt 1648
Gln Val Lys Asn Gly Gly Ile Tyr Glu Gly Val Phe Lys Thr Tyr Ser
        270                 275                 280

ccg aag tgt gat ttg gta ctt gat gcc gca cat gag aaa agt aca gaa 1696
Pro Lys Cys Asp Leu Val Leu Asp Ala Ala His Glu Lys Ser Thr Glu
    285                 290                 295

tcc agt tcg ggg ccg aaa cgt gaa gaa ata atg gag agt att ttg ttc 1744
Ser Ser Ser Gly Pro Lys Arg Glu Glu Ile Met Glu Ser Ile Leu Phe
300                 305                 310                 315

aaa tgt tca gac ttt gtt gtg gta cag ttt aaa gat atg gac tcc agt 1792
Lys Cys Ser Asp Phe Val Val Val Gln Phe Lys Asp Met Asp Ser Ser
                320                 325                 330

tat gca aaa aga gat gct ttt act gac tct gct atc agt gct aaa gtg 1840
Tyr Ala Lys Arg Asp Ala Phe Thr Asp Ser Ala Ile Ser Ala Lys Val
            335                 340                 345

aat ggc gaa cac aaa gag aag gac ctg gag ccc tgg gat gca ggt gaa 1888
Asn Gly Glu His Lys Glu Lys Asp Leu Glu Pro Trp Asp Ala Gly Glu
        350                 355                 360

ctc aca gcc aat gag gaa ctt gag gct ttg gaa aat gac gta tct aat 1936
Leu Thr Ala Asn Glu Glu Leu Glu Ala Leu Glu Asn Asp Val Ser Asn
    365                 370                 375

gga tgg gat ccc aat gat atg ttt cga tat aat gaa gaa aat tat ggt 1984
Gly Trp Asp Pro Asn Asp Met Phe Arg Tyr Asn Glu Glu Asn Tyr Gly
380                 385                 390                 395

gta gtg tct acg tat gat agc agt tta tct tcg tat aca gtg ccc tta 2032
Val Val Ser Thr Tyr Asp Ser Ser Leu Ser Ser Tyr Thr Val Pro Leu
                400                 405                 410

gaa aga gat aac tca gaa gaa ttt tta aaa cgg gaa gca agg gca aac 2080
Glu Arg Asp Asn Ser Glu Glu Phe Leu Lys Arg Glu Ala Arg Ala Asn
            415                 420                 425

cag tta gca gaa gaa att gag tca agt gcc cag tac aaa gct cga gtg 2128
Gln Leu Ala Glu Glu Ile Glu Ser Ser Ala Gln Tyr Lys Ala Arg Val
        430                 435                 440

gcc ctg gaa aat gat gat agg agt gag gaa gaa aaa tac aca gca gtt 2176
Ala Leu Glu Asn Asp Asp Arg Ser Glu Glu Glu Lys Tyr Thr Ala Val
    445                 450                 455

cag aga aat tcc agt gaa cgt gag ggg cac agc ata aac act agg gaa 2224
Gln Arg Asn Ser Ser Glu Arg Glu Gly His Ser Ile Asn Thr Arg Glu
460                 465                 470                 475

aat aaa tat att cct cct gga caa aga aat aga gaa gtc ata tcc tgg 2272
Asn Lys Tyr Ile Pro Pro Gly Gln Arg Asn Arg Glu Val Ile Ser Trp
                480                 485                 490

gga agt ggg aga cag aat tca ccg cgt atg ggc cag cct gga tcg ggc 2320
Gly Ser Gly Arg Gln Asn Ser Pro Arg Met Gly Gln Pro Gly Ser Gly
            495                 500                 505

tcc atg cca tca aga tcc act tct cac act tca gat ttc aac ccg aat 2368
Ser Met Pro Ser Arg Ser Thr Ser His Thr Ser Asp Phe Asn Pro Asn
        510                 515                 520

tct ggt tca gac caa aga gta gtt aat gga ggt gtt ccc tgg cca tcg 2416
Ser Gly Ser Asp Gln Arg Val Val Asn Gly Gly Val Pro Trp Pro Ser
    525                 530                 535

cct tgc cca tct cct tcc tct cgc cca cct tct cgc tac cag tca ggt 2464
Pro Cys Pro Ser Pro Ser Ser Arg Pro Pro Ser Arg Tyr Gln Ser Gly
540                 545                 550                 555

ccc aac tct ctt cca cct cgg gca gcc acc cct aca cgg ccg ccc tcc 2512
Pro Asn Ser Leu Pro Pro Arg Ala Ala Thr Pro Thr Arg Pro Pro Ser
                560                 565                 570

agg ccc ccc tcg cgg cca tcc aga ccc ccg tct cac ccc tct gct cat 2560
Arg Pro Pro Ser Arg Pro Ser Arg Pro Pro Ser His Pro Ser Ala His
            575                 580                 585

ggt tct cca gct cct gtc tct act atg cct aaa cgc atg tct tca gaa 2608
Gly Ser Pro Ala Pro Val Ser Thr Met Pro Lys Arg Met Ser Ser Glu
        590                 595                 600

ggg cct cca agg atg tcc cca aag gcc cag cga cat cct cga aat cac 2656
Gly Pro Pro Arg Met Ser Pro Lys Ala Gln Arg His Pro Arg Asn His
    605                 610                 615

aga gtt tct gct ggg agg ggt tcc ata tcc agt ggc cta gaa ttt gta 2704
Arg Val Ser Ala Gly Arg Gly Ser Ile Ser Ser Gly Leu Glu Phe Val
620                 625                 630                 635

tcc cac aac cca ccc agt gaa gca gct act cct cca gta gca agg acc 2752
Ser His Asn Pro Pro Ser Glu Ala Ala Thr Pro Pro Val Ala Arg Thr
                640                 645                 650

agt ccc tcg ggg gga acg tgg tca tca gtg gtc agt ggg gtt cca aga 2800
Ser Pro Ser Gly Gly Thr Trp Ser Ser Val Val Ser Gly Val Pro Arg
            655                 660                 665

tta tcc cct aaa act cat aga ccc agg tct ccc aga cag aac agt att 2848
Leu Ser Pro Lys Thr His Arg Pro Arg Ser Pro Arg Gln Asn Ser Ile
        670                 675                 680

gga aat acc ccc agt ggg cca gtt ctt gct tct ccc caa gct ggt att 2896
Gly Asn Thr Pro Ser Gly Pro Val Leu Ala Ser Pro Gln Ala Gly Ile
    685                 690                 695

att cca act gaa gct gtt gcc atg cct att cca gct gca tct cct acg 2944
Ile Pro Thr Glu Ala Val Ala Met Pro Ile Pro Ala Ala Ser Pro Thr
700                 705                 710                 715

cct gct agt cct gca tcg aac aga gct gtt acc cct tct agt gag gct 2992
Pro Ala Ser Pro Ala Ser Asn Arg Ala Val Thr Pro Ser Ser Glu Ala
                720                 725                 730

aaa gat tcc agg ctt caa gat cag agg cag aac tct cct gca ggg aat 3040
Lys Asp Ser Arg Leu Gln Asp Gln Arg Gln Asn Ser Pro Ala Gly Asn
            735                 740                 745

aaa gaa aat att aaa ccc aat gaa aca tca cct agc ttc tca aaa gct 3088
Lys Glu Asn Ile Lys Pro Asn Glu Thr Ser Pro Ser Phe Ser Lys Ala
        750                 755                 760

gaa aac aaa ggt ata tca cca gtt gtt tct gaa cat aga aaa cag att 3136
Glu Asn Lys Gly Ile Ser Pro Val Val Ser Glu His Arg Lys Gln Ile
    765                 770                 775

gat gat tta aag aaa ttt aag aat gat ttt agg tta cag cca agt tct 3184
Asp Asp Leu Lys Lys Phe Lys Asn Asp Phe Arg Leu Gln Pro Ser Ser
780                 785                 790                 795

act tct gaa tct atg gat caa cta cta aac aaa aat aga gag gga gaa 3232
Thr Ser Glu Ser Met Asp Gln Leu Leu Asn Lys Asn Arg Glu Gly Glu
                800                 805                 810

aaa tca aga gat ttg atc aaa gac aaa att gaa cca agt gct aag gat 3280
Lys Ser Arg Asp Leu Ile Lys Asp Lys Ile Glu Pro Ser Ala Lys Asp
            815                 820                 825

tct ttc att gaa aat agc agc agc aac tgt acc agt ggc agc agc aag 3328
Ser Phe Ile Glu Asn Ser Ser Ser Asn Cys Thr Ser Gly Ser Ser Lys
        830                 835                 840

ccg aat agc ccc agc att tcc cct tca ata ctt agt aac acg gag cac 3376
Pro Asn Ser Pro Ser Ile Ser Pro Ser Ile Leu Ser Asn Thr Glu His
    845                 850                 855

aag agg gga cct gag gtc act tcc caa ggg gtt cag act tcc agc cca 3424
Lys Arg Gly Pro Glu Val Thr Ser Gln Gly Val Gln Thr Ser Ser Pro
860                 865                 870                 875

gca tgt aaa caa gag aaa gac gat aag gaa gag aag aaa gac gca gct 3472
Ala Cys Lys Gln Glu Lys Asp Asp Lys Glu Glu Lys Lys Asp Ala Ala
                880                 885                 890

gag caa gtt agg aaa tca aca ttg aat ccc aat gca aag gag ttc aac 3520
Glu Gln Val Arg Lys Ser Thr Leu Asn Pro Asn Ala Lys Glu Phe Asn
            895                 900                 905

cca cgt tcc ttc tct cag cca aag cct tct act acc cca act tca cct 3568
Pro Arg Ser Phe Ser Gln Pro Lys Pro Ser Thr Thr Pro Thr Ser Pro
        910                 915                 920

cgg cct caa gca caa cct agc cca tct atg gtg ggt cat caa cag cca 3616
Arg Pro Gln Ala Gln Pro Ser Pro Ser Met Val Gly His Gln Gln Pro
    925                 930                 935

act cca gtt tat act cag cct gtt tgt ttt gca cca aat atg atg tat 3664
Thr Pro Val Tyr Thr Gln Pro Val Cys Phe Ala Pro Asn Met Met Tyr
940                 945                 950                 955

cca gtc cca gtg agc cca ggc gtg caa cct tta tac cca ata cct atg 3712
Pro Val Pro Val Ser Pro Gly Val Gln Pro Leu Tyr Pro Ile Pro Met
                960                 965                 970

acg ccc atg cca gtg aat caa gcc aag aca tat aga gca gta cca aat 3760
Thr Pro Met Pro Val Asn Gln Ala Lys Thr Tyr Arg Ala Val Pro Asn
            975                 980                 985

atg ccc caa cag cgg caa gac cag cat cat cag agt gcc atg atg cac 3808
Met Pro Gln Gln Arg Gln Asp Gln His His Gln Ser Ala Met Met His
        990                 995                 1000

cca gcg tca gca gcg ggc cca ccg att gca gcc acc cca cca gct 3853
Pro Ala Ser Ala Ala Gly Pro Pro Ile Ala Ala Thr Pro Pro Ala
    1005                1010                1015

tac tcc acg caa tat gtt gcc tac agt cct cag cag ttc cca aat 3898
Tyr Ser Thr Gln Tyr Val Ala Tyr Ser Pro Gln Gln Phe Pro Asn
    1020                1025                1030

cag ccc ctt gtt cag cat gtg cca cat tat cag tct cag cat cct 3943
Gln Pro Leu Val Gln His Val Pro His Tyr Gln Ser Gln His Pro
    1035                1040                1045

cat gtc tat agt cct gta ata cag ggt aat gct aga atg atg gca 3988
His Val Tyr Ser Pro Val Ile Gln Gly Asn Ala Arg Met Met Ala
    1050                1055                1060

cca cca aca cac gcc cag cct ggt tta gta tct tct tca gca act 4033
Pro Pro Thr His Ala Gln Pro Gly Leu Val Ser Ser Ser Ala Thr
    1065                1070                1075

cag tac ggg gct cat gag cag acg cat gcg atg tat gca tgt ccc 4078
Gln Tyr Gly Ala His Glu Gln Thr His Ala Met Tyr Ala Cys Pro
    1080                1085                1090

aaa tta cca tac aac aag gag aca agc cct tct ttc tac ttt gcc 4123
Lys Leu Pro Tyr Asn Lys Glu Thr Ser Pro Ser Phe Tyr Phe Ala
    1095                1100                1105

att tcc acg ggc tcc ctt gct cag cag tat gcg cac cct aac gct 4168
Ile Ser Thr Gly Ser Leu Ala Gln Gln Tyr Ala His Pro Asn Ala
    1110                1115                1120

acc ctg cac cca cat act cca cac cct cag cct tca gct acc ccc 4213
Thr Leu His Pro His Thr Pro His Pro Gln Pro Ser Ala Thr Pro
    1125                1130                1135

act gga cag cag caa agc caa cat ggt gga agt cat cct gca ccc 4258
Thr Gly Gln Gln Gln Ser Gln His Gly Gly Ser His Pro Ala Pro
    1140                1145                1150

agt cct gtt cag cac cat cag cac cag gcc gcc cag gct ctc cat 4303
Ser Pro Val Gln His His Gln His Gln Ala Ala Gln Ala Leu His
    1155                1160                1165

ctg gcc agt cca cag cag cag tca gcc att tac cac gcg ggg ctt 4348
Leu Ala Ser Pro Gln Gln Gln Ser Ala Ile Tyr His Ala Gly Leu
    1170                1175                1180

gcg cca act cca ccc tcc atg aca cct gcc tcc aac acg cag tcg 4393
Ala Pro Thr Pro Pro Ser Met Thr Pro Ala Ser Asn Thr Gln Ser
    1185                1190                1195

cca cag aat agt ttc cca gca gca caa cag act gtc ttt acg atc 4438
Pro Gln Asn Ser Phe Pro Ala Ala Gln Gln Thr Val Phe Thr Ile
    1200                1205                1210

cat cct tct cac gtt cag ccg gcg tat acc aac cca ccc cac atg 4483
His Pro Ser His Val Gln Pro Ala Tyr Thr Asn Pro Pro His Met
    1215                1220                1225

gcc cac gta cct cag gct cat gta cag tca gga atg gtt cct tct 4528
Ala His Val Pro Gln Ala His Val Gln Ser Gly Met Val Pro Ser
    1230                1235                1240

cat cca act gcc cat gcg cca atg atg cta atg acg aca cag cca 4573
His Pro Thr Ala His Ala Pro Met Met Leu Met Thr Thr Gln Pro
    1245                1250                1255

ccc ggc ggt ccc cag gcc gcc ctc gct caa agt gca cta cag ccc 4618
Pro Gly Gly Pro Gln Ala Ala Leu Ala Gln Ser Ala Leu Gln Pro
    1260                1265                1270

att cca gtc tcg aca aca gcg cat ttc ccc tat atg acg cac cct 4663
Ile Pro Val Ser Thr Thr Ala His Phe Pro Tyr Met Thr His Pro
    1275                1280                1285

tca gta caa gcc cac cac caa cag cag ttg taa ggc tgc cct gga 4708
Ser Val Gln Ala His His Gln Gln Gln Leu Gly Cys Pro Gly
    1290                1295                1300

gga acc gaa agg cca aat tcc ctc ctc cct tct act gct tct acc 4753
Gly Thr Glu Arg Pro Asn Ser Leu Leu Pro Ser Thr Ala Ser Thr
        1305                1310                1315

aac tgg aag cac aga aaa cta gaa ttt cat tta ttt tgt ttt taa 4798
Asn Trp Lys His Arg Lys Leu Glu Phe His Leu Phe Cys Phe
        1320                1325                1330

aat ata tat gtt  gat ttc ttg taa cat cca  ata gga atg cta aca        4843
Asn Ile Tyr Val  Asp Phe Leu     His Pro  Ile Gly Met Leu Thr
            1335                     1340                 1345

gtt cac ttg cag tgg  aag ata ctt gga ccg  agt aga ggc att tag        4888
Val His Leu Gln Trp  Lys Ile Leu Gly Pro  Ser Arg Gly Ile
                1350                 1355

gaa  ctt ggg ggc tat tcc  ata att cca tat gct  gtt tca gag tcc       4933
Glu  Leu Gly Gly Tyr Ser  Ile Ile Pro Tyr Ala  Val Ser Glu Ser
1360                 1365                 1370

cgc  agg tac ccc agc tct  gct tgc cga aac tgg  aag tta ttt att       4978
Arg  Arg Tyr Pro Ser Ser  Ala Cys Arg Asn Trp  Lys Leu Phe Ile
1375                 1380                 1385

ttt  taa taa ccc ttg aaa gtc atg  aac aca tca gct agc                5017
Phe          Pro Leu Lys Val Met  Asn Thr Ser Ala Ser
1390                         1395                 1400